# EUROPEAN PATENT APPLICATION

(11) **EP 4 674 406 A1**
(43) Date of publication of application: **07.01.2026**
(21) Application number: 24185879.4
(22) Date of filing: 02.07.2024
(51) Int. Cl.: A61K 8/67, A61K 8/73, A61K 8/9789, A61Q 5/00, A61Q 7/00

(54) **GENESIS HSP3 - A UNIQUE COMPLEX OF ACTIVE INGREDIENTS FOR HAIR CARE**

(71) Applicant: Hdrey Laboratories Sp. z o.o., 00-710 Warszawa (PL)
(72) Inventor: The designation of the inventor has not yet been filed

(57) **Abstract**

GENESIS HSP3 - a unique complex of active ingredients providing hydration, regeneration, and protection for hair. To achieve the best results in improving the appearance and condition of hair, an active complex of multi-functional ingredients was designed and created.
H - Hyaluronic Acid INCI: Sodium Hyaluronate
P - Peptide INCI: Biotinoyl Tripeptide-1
S - Stem Cells INCI: Olea Europaea Callus Culture Lysate

As a result of combining the well-researched ingredients described above, the complex provides the following benefits:
- Hydration of hair and scalp
- Nourishment of hair
- Increased hair volume
- Easier detangling
- Reduction of hair loss
- Color protection for hair
- Protection of hair from harmful external factors

The use of this complex in cosmetic hair care products will result in safe and highly effective products.

## Description

GENESIS HSP3 - a unique complex of active ingredients providing hydration, regeneration, and protection for hair. To achieve the best results in improving the appearance and condition of hair, an active complex of multi-functional ingredients was designed and created.

### H - Hyaluronic Acid INCI: Sodium Hyaluronate

Hyaluronic acid in a form designed for effective hydration of hair and scalp. Research has shown its effectiveness in moisturizing and nourishing hair, as well as soothing redness and itching of the scalp. Hyaluronic acid helps alleviate dandruff and makes hair easier to comb, reducing the amount of hair lost during combing. Combining hyaluronic acid with cationic guar gum further enhances its detangling properties.

According to the knowlege described in the literature hyaluronic acid has a broad range of cosmetic and therapeutic applications due to its unique physicochemical properties and involvement in various essential biological processes, including cell signaling, wound reparation, and tissue regeneration. [1] Non crosslinked hyaluronic acid has a dual action in the experimental conditions tested: it can protect dermal papilla cells from oxidative stress and increase the vascular endothelial growth factor levels.[2] It has also been tested that the addition of hyaluronic acid to preparations that create a film on the hair strengthens the protective coating and increases its stability.[3]

### P - Peptide INCI: Biotinoyl Tripeptide-1

A tripeptide combining vitamin H with the matrikine family - signaling peptides derived from extracellular matrix proteins, GHK. The GHK peptide is a well-studied active ingredient known for its exceptional biological activity, impacting skin cell receptors. This peptide, combined with vitamin H (biotin), can increase the synthesis of extracellular matrices such as collagen IV and laminin 5. It slows down the aging of hair follicles and improves their structure by enhancing microcirculation in the scalp area. It positively affects the anchoring of hair in the skin follicles, preventing hair loss. It can activate the expression of tissue repair genes, promoting the reconstruction and repair of skin structure. It also supports cell proliferation and differentiation and stimulates hair growth.

Research described in the literature shows that cosmetics containing peptides effectively improve the condition of hair, affecting, among other things, its structure and mechanical resistance. [4] Peptides and amino acids are well-researched substances that influence the condition of both skin and hair [5].

### S - Stem Cells INCI: Olea Europaea Callus Culture Lysate

An active ingredient derived from the stem cells of the wild olive tree. Inspired by architecture, it provides a new, holistic approach to hair care, considering the scalp, hair follicle, and hair shaft. The combined action on each of these parts effectively improves hair condition.

It protects hair from the harmful effects of high temperatures and UV radiation - up to 70% better than placebo products. It ensures color protection for dyed hair. It hydrates and nourishes hair, increasing hair diameter by up to 14%, while also providing softness and shine. Studies have shown that this ingredient increases the expression of key anchoring proteins' genes, strengthening the connection between the scalp and hair follicle. Additionally, it acts on the scalp - moisturizing it, reducing redness, and soothing irritation.

Studies described in the literature indicate that preparations obtained from olives contain substances with strong antioxidant properties. [6] Cosmetic products containing olive leaf and fruit extracts have anti-inflammatory and hair-strengthening properties. [7]

As a result of combining the well-researched ingredients described above, the complex provides the following benefits:
- Hydration of hair and scalp
- Nourishment of hair
- Increased hair volume
- Easier detangling
- Reduction of hair loss
- Color protection for hair
- Protection of hair from harmful external factors

The use of this complex in cosmetic hair care products will result in safe and highly effective products.

### REFERENCES:

[1] Al-Halaseh LK, Al-Jawabri NA, Tarawneh SK, Al-Qdah WK, Abu-Hajleh MN, Al-Samydai AM, Ahmed MA. "A review of the cosmetic use and potentially therapeutic importance of hyaluronic acid." J Appl Pharm Sci, 2022; 12(07):034-041.
[2] Zerbinati, Nicola, et al. "In vitro hair growth promoting effect of a noncrosslinked hyaluronic acid in human dermal papilla cells." BioMed Research International 2021.1 (2021): 5598110.
[3] Sionkowska, Alina, et al. "Preparation and characterization of collagen/chitosan/hyaluronic acid thin films for application in hair care cosmetics." Pure and Applied Chemistry 89.12 (2017): 1829-1839.
[4] Tinoco, Ana, et al. "Biotechnology of functional proteins and peptides for hair cosmetic formulations." Trends in Biotechnology 40.5 (2022): 591-605.
[5] Ahsan, Haseeb. "Immunopharmacology and immunopathology of peptides and proteins in personal products." Journal of Immunoassay and Immunochemistry 40.4 (2019): 439-447.
[6] Al-Owamri, Fatma Said Nasser, et al. "Phytochemical, Antioxidant, hair growth and wound healing property of Juniperus excelsa, Olea oleaster and Olea europaea." Journal of King Saud University-Science 35.2 (2023): 102446.
[7] Yateem, Hiba, Michel Hanania, and Nida Mosleh. "Formulation and evaluation of herbal shampoo containing olive leaves extract." (2018).

## Claims

1. qualitative composition of the complex of active ingredients GENESIS HSP3
Hyaluronic Acid (Sodium Hyaluronate),
Peptide (Biotinoyl Tripeptide-1),
Stem Cells (Olea Europaea Callus Culture Lysate)

2. quantitative composition of the complex of active ingredients GENESIS HSP3

3. name of the complex of active ingredients GENESIS HSP3
